# EUROPEAN PATENT SPECIFICATION

(11) **EP 1 588 732 B1**
(45) Date of publication and mention of the grant of the patent: **13.06.2007**
(21) Application number: 04009580.4
(22) Date of filing: 22.04.2004
(51) Int. Cl.: A61M 25/10, A61K 9/00

(54) **Extractable filler for inserting medicine into animal tissue**
Extrahierbare Abfüllvorrichtung zum Einsetzen von Arzneimittel ins tierische Gewebe
Dispositif de remplissage extractible servant à insérer du médicament dans un tissu animal

(43) Date of publication of application: 26.10.2005
(73) Proprietor: Lin, Kwan-Ku, Pasadena, CA 91105 (US); Yuan, Philip S., Fayetteville, NY 13066 (US)
(72) Inventor: Lin, Kwan-Ku, Pasadena, CA 91105 (US); Yuan, Philip S., Fayetteville, NY 13066 (US)
(74) Representative: Tergau & Pohl Patentanwälte

(56) References cited:
- US-A- 4 148 871
- US-A- 4 774 091
- US-A1- 2001 047 157
- US-A1- 2002 177 866

## Description

### FIELD OF THE INVENTION

The present invention relates generally to an instrument which is used in the restorative operation of animal tissue disorder. More specifically this surgical instrument can be used to insert a medical material into an animal tissue such that the instrument can be separated from the medical material and drawn out of the animal tissue so as to avert the possibility of tissue rejection. The closest prior art is US 2002/177866 A, which defines the preamble of claim 1.

### BACKGROUND OF THE INVENTION

The surgical treatment of animal tissue disorder can be generally attained by one of three methods, which include the hypodermic injection of medicine, the balloon-insertion of medicine, and the filler-insertion of medicine. For example, the U.S. Patent Nos. 5,972,105; 6,066,154; and 6,248,110B1 disclose respectively a method for treating bone tissue disorders, such as osteoporosis and vertebral compression fractures. The method involves the use of a balloon (made by the Kyphon Crop., U.S.A.) by which the tissue is expanded to facilitate the inserting of the medicine. This balloon method is defective in design in that the medicine is apt to spread aimlessly in the tissue without boundary. Without containment, the medicine is not as effective and there is the possibility of injury to the surrounding tissues.

In order to prevent the drawbacks of the balloon method described above, the filler-insertion method is used to implant the medicine in animal tissue in such a way that the medicine is contained in the filler, and that both the medicine and the filler are implanted in the animal tissue. This filler-insertion method is often carried out in danger of the tissue rejection of the filler.

### SUMMARY OF THE INVENTION

The present invention provides an extractable device for inserting a medicinal filling into an animal tissue, said device comprising:
a filling member comprising a flexible and hermetic wall and provided with a holding portion, an injection port at one end of the holding portion, and an opening at another end of the holding portion;
one or more thread, each having one end for fastening releasably said opening of said holding portion in such a manner that said opening is leakproof; and
a pasty medicine to be injected into said holding portion via said injection port of said filling member in the wake of a process for inserting said filling member into the animal tissue whereby said pasty medicine solidifies in said holding portion of said filling member;
said opening of said holding portion being unfastened at the time when other end of said threads is pulled by an external force, thereby enabling said filling member to be extracted from the animal tissue so as to leave only said medicine in the animal tissue.

Preferably, said holding portion of said filling member is integrally formed by said flexible and hermetic wall into a body in the form of sac, bag, or ball.

Preferably, said pasty medicine is a mixture of a liquid and a medicinal powdered substance or medicinal granular substance.

Preferably, the device of the present invention further comprises an injection tool for injecting said pasty medicine into said holding portion via said injection port.

Preferably, said injection tool comprises a guide tube and a syringe, wherein one end of said guide tube is connected to said injection port of said filling member and another end of said guide tube is connected to said syringe in which said pasty medicine is held, so that said pasty medicine is able to be injected into said holding portion of said filling member by said syringe via said injection port and said guide tube.

Preferably, said flexible and hermetic wall is a double-layer tubular wall having one end of an inner layer thereof being provided with said injection port of said holding portion, and having another end thereof being a folded double-layer end with said opening of said holding portion, wherein said medicine is released from said filling member by pulling a free end of an outer layer of the double-layer tubular wall to retreat the folded double-layer end, after said opening of said holding portion being unfastened.

Preferably, said one or more thread is between said inner layer and said outer layer of said double-layer tubular wall.

The flexible wall of the filling member of the present invention is hermetic and made of a biocompatible or biosynthetic material, such as rubber, elastic plastic, titanium, goat intestine, and the like. The flexible wall can be formed into an object in the form of sac, bag, ball, cylinder or rectangular column integrally or by joining separate pieces.

The filling member of the present invention may contain a ray imaging material, such as a metal wire, by which the precise position of the filling member can be easily located by a ray imaging system, such as an X-ray machine.

The features and the advantages of the present invention will be more readily understood upon a thoughtful deliberation of the following detailed description of the preferred embodiments of the present invention with reference to the accompanying drawings.

### BRIEF DESCRIPTION OF THE DRAWINGS

FIG. 1 shows a sectional schematic view of the present invention.
FIGS. 2a-2c are schematic views illustrating the lashing of the opening of the holding portion of the filling member of the present invention.
FIGS. 3a and 3b are sectional schematic view of the present invention at work.
FIGS. 4a-4c are schematic views illustrating the unlashing of the opening of the holding portion of the filling member of the present invention upon completion of the injection of the medicine into the holding portion of the filling member.
FIG. 5a shows a schematic view of the connection tube of the implantation-injection apparatus of the present invention.
FIGS. 5b and 5c are sectional schematic views illustrating the process in which the filling member of the present invention is extracted from the animal tissue.
FIGS. 6a and 6b are schematic views illustrating that the opening of the holding portion of the filling member of the present invention is releasably lashed by a thread.
FIGS. 7a-7d are schematic views illustrating a process in which the opening of the holding portion of the filling member of the present invention is releasably lashed by two threads in conjunction with sewing.
FIG. 8a is a schematic view illustrating a process in which a double-layer wall of the holding portion of the filling member of the present invention is formed.
FIG. 8b illustrating a process in which the double-layer wall of the holding portion of the filling member of the present invention is retreated from the solidified medicine.

### DETAILED DESCRIPTION OF THE PREFERRED EMBODIMENTS

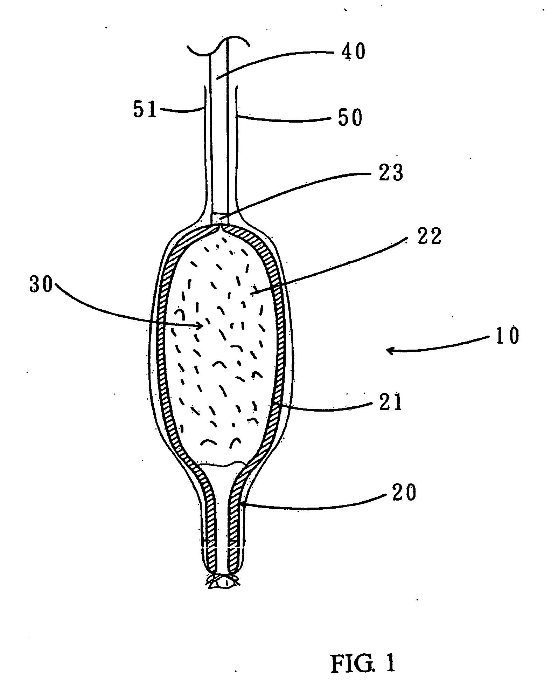

As shown in FIG. 1, an extractable filler 10 embodied in the present invention comprises a filling member 20, a pasty medicine 30, a connection tube 40, and two threads 50 and 51. The filling member 20 is formed of a flexible wall 21 and is provided with a holding portion 22 and an injection port 23. The pasty medicine 30 is injected into the holding portion 22 via the connection tube 40 and the injection port 23. The dotted line 3-3 shows a direction in which a section of the filling member 20 is taken.

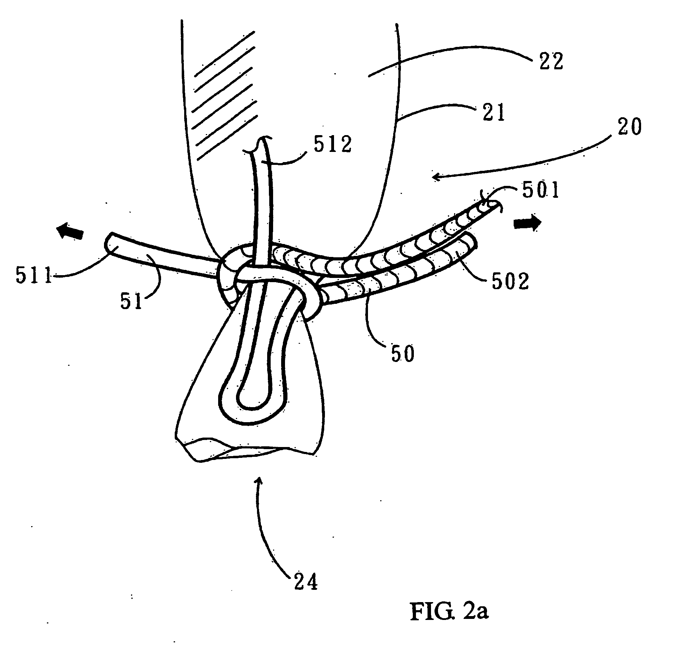

As shown in FIG. 2a, the holding portion 22 of the filling member 20 is integrally formed of the hermetic flexible wall 21, and is provided with an opening 24 opposite to the injection port 23 of the filling member 20. The filling member 20, for example, is an inflatable rubber balloon with an additional opening like the opening 24. The opening 24 is lashed by two threads 50 and 51. The first thread 50 has a first end 501 and a second end 502, while the second thread 51 has a first end 511 and a second end 512. The two threads 50 and 51 are in fact fastened releasably to the flexible wall 21 near the opening 24. The way by which they are fastened together is not shown in the drawing.

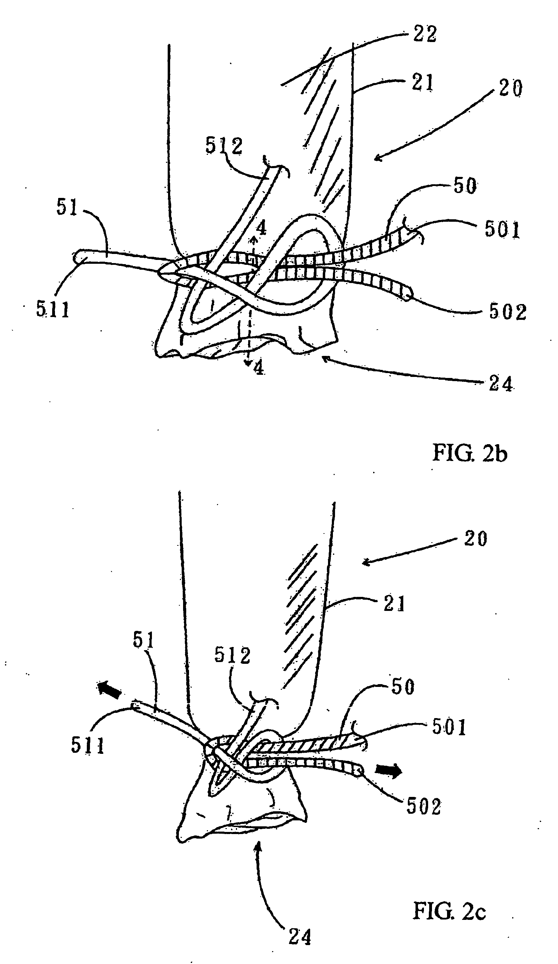

The opening 24 of the holding portion 22 of the filling member 20 is securely tied up to prevent the medicine 30 from leaking out of the holding portion 22 by means of the two threads 50 and 51 which are releasably entangled in such a manner that the first end 511 of the second thread 51 is wound around the first thread 50. Upon completion of the winding process, the flexible wall 21 surrounding the opening 24 is located in a position between the two threads 50 and 51, as indicated by a dotted line 4-4 in FIG. 2b. Thereafter, both ends 501 and 502 of the first thread 50, and the first end 511 of the second thread 51 are respectively pulled rightward and leftwards at the same time, as illustrated in FIG. 2c. As a result, the opening 24 of the filling member 20 is leakproof.

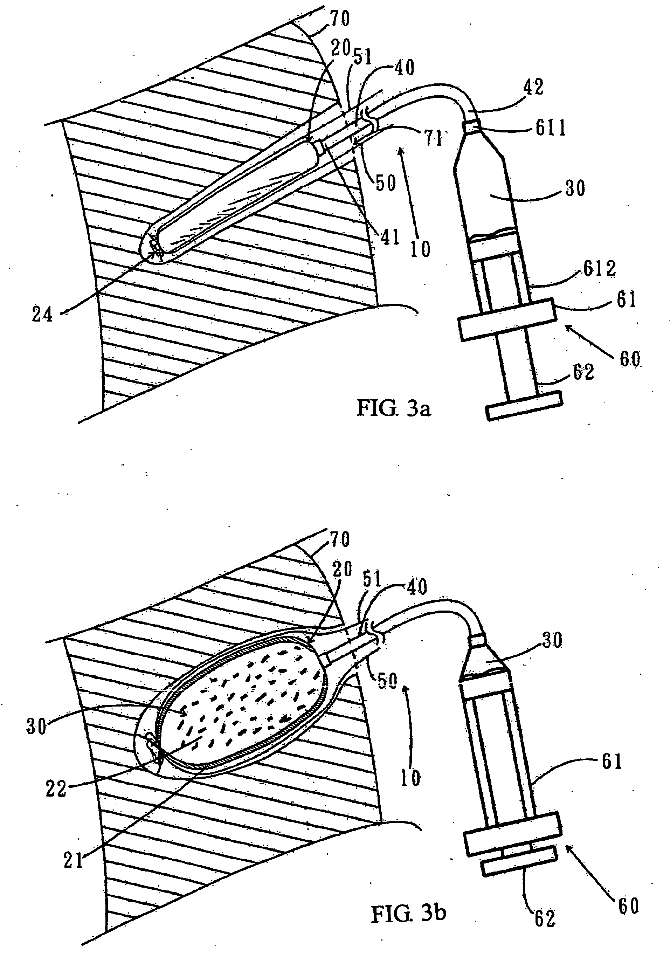

As shown in FIGS. 3a and 3b, the filling member 20 is first inserted into a blind hole 71 of an animal tissue 70. The pasty medicine 30 is then injected into the holding portion 22 of the filling member 20 by a syringe 60 in conjunction with the connection tube 40. The filling member 20 is thus inflated by the medicine 30, as shown in FIG. 3b. The connection tube 40 has one end 41 which is connected with the filling member 20, and another end 42 which is connected to one end 611 of a barrel 61 of the syringe 60. A plunger 62 is slidably inserted into another end 612 of the barrel 61 in which the pasty medicine 30 is contained.

The pasty medicine 30 is a mixture of a liquid and one or more kinds of animal tissue drugs in the form of powder, granule, or colloid. The pasty medicine 30 is capable of solidification.

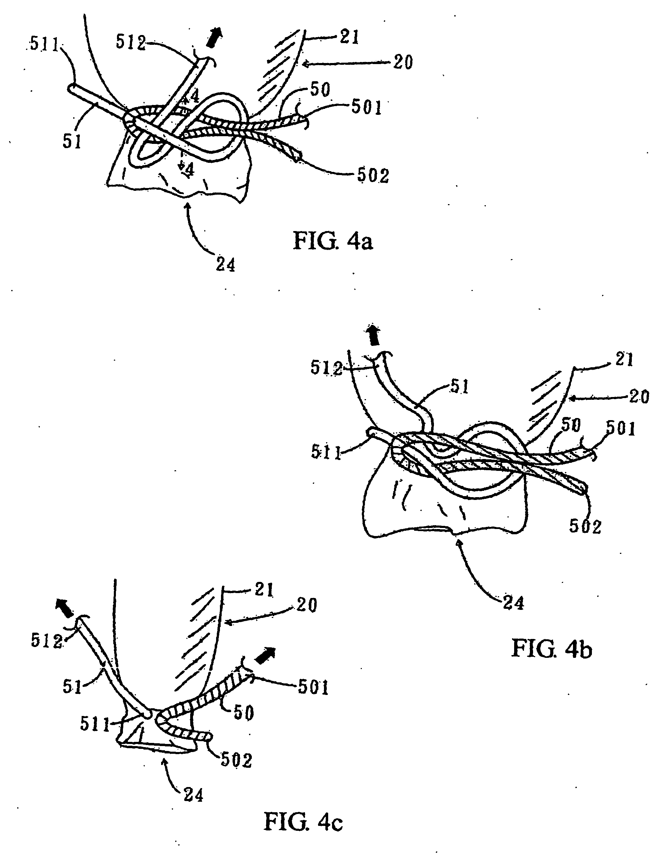

Upon completion of the solidification of the pasty medicine 30 in the blind hole 71 of the animal tissue 70, the filling member 20 must be extracted from the blind hole 71 of the animal tissue 70, so as to leave only the medicine 30 in the blind hole 71 of the animal tissue 70 to prevent the rejection of the filling member 20 by the animal tissue 70. The extraction of the filling member 20 from the blind hole 71 of the animal tissue 70 involves a first step in which the second end 512 of the second thread 51 is pulled upward as indicated by an arrow in FIG. 4a. As a result, the two threads 50 and 51 become loosened, as shown in FIG. 4b. Thereafter, the first end 501 of the first thread 50 and the second end 512 of the second thread 51 are respectively pulled in a direction away from the opening 24 of the filling member 20, as illustrated in FIG. 4c. The opening 24 is thus unfastened completely.

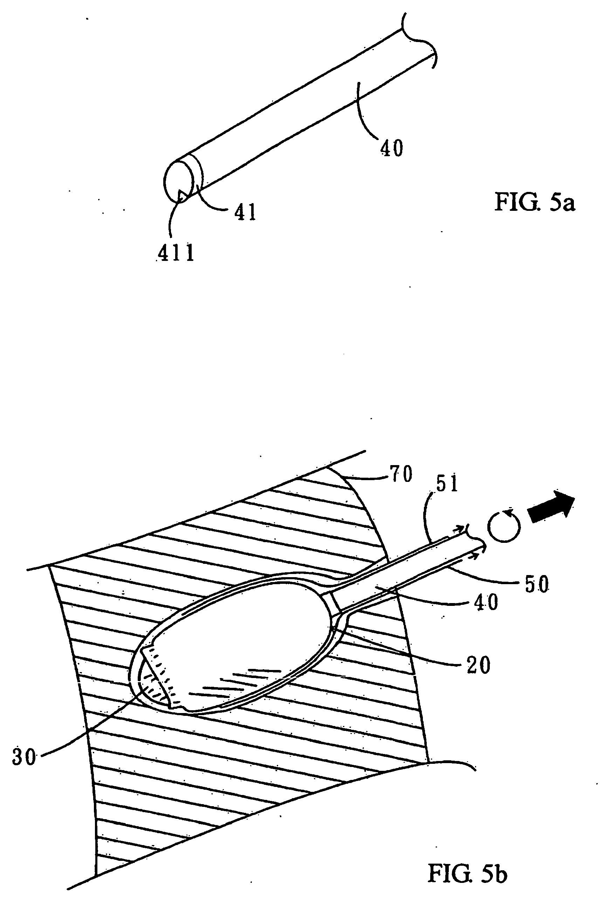

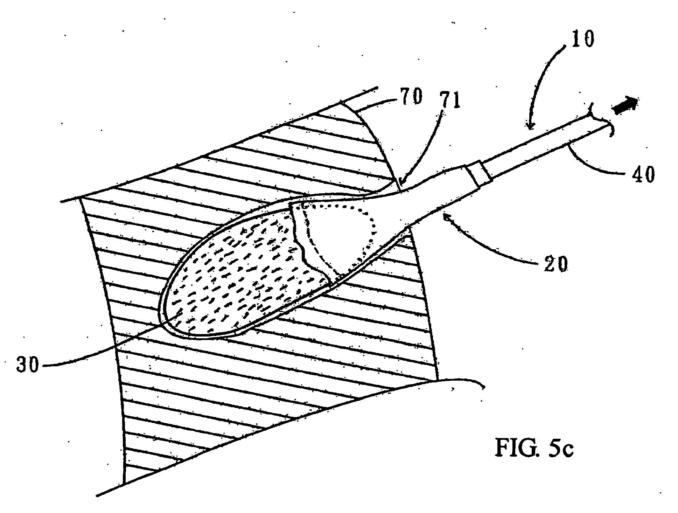

As shown in FIG. 5a, the connection tube 40 is provided in one end 41 with a pointed projection 411 inside the tube. As the connection tube 40 is slightly twisted, the solidified medicine 30 is severed by the pointed projection 411 of the connection tube 40. The filling member 20 can be drawn out of the blind hole 71 of the animal tissue 70 by the connection tube 40, as illustrated in FIG. 5b and FIG. 5c. As a result, only the medicine 30 is left in the blind hole 71 of the animal tissue.

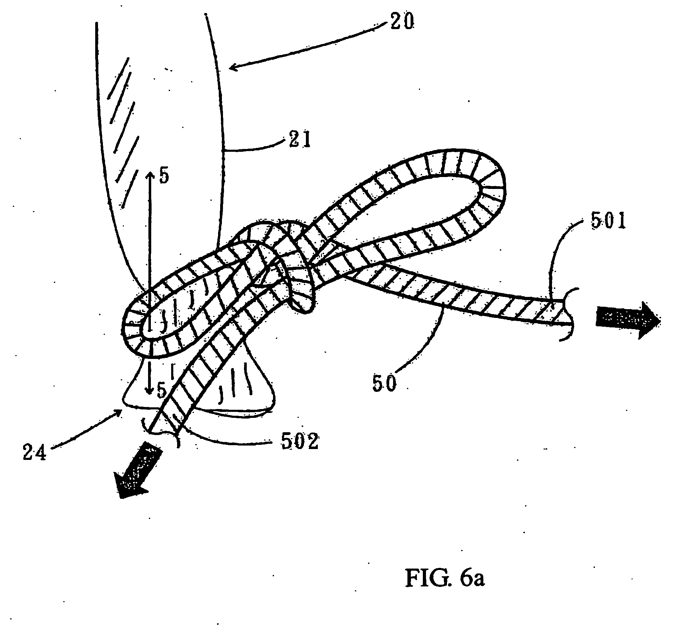

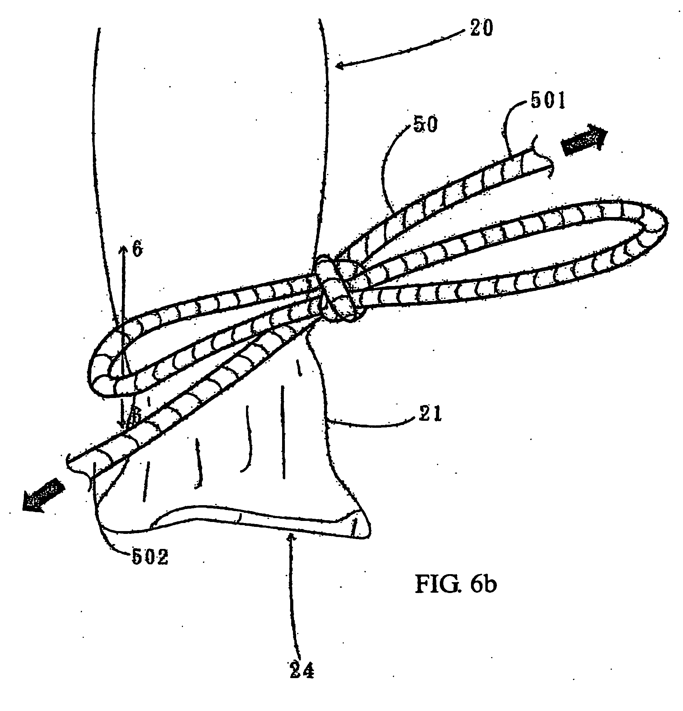

The opening 24 of the filling member 20 may be fastened by only one thread 50, as illustrated in FIGS. 6a and 6b. The thread 50 has a first end 501 and a second end 502. With the thread 50, a knot is formed to lash the opening 24 of the filling member 20 in such a manner that the flexible wall 21 of the opening 24 is surrounded by a loop as indicated by a line 5-5 in FIG. 6a. With the second end 502 of the thread 50 remaining in the stationary state, the first end 501 is pulled to fasten the opening 24. The opening 24 is unfastened by pulling the second end 502 of the thread 20, thereby resulting in separation of the filling member 20 from the thread 50.

The thread 50 can be also used to form a different knot, as shown in FIG. 6b. The flexible wall 21 of the opening 24 of the filling member 20 is surrounded by a loop as indicated by a line 6-6 in FIG. 6b. As the first end 501 of the thread 50 is pulled in a direction away from the filling member 20, the opening 24 of the filling member 20 is lashed to become leakproof. The opening 24 of the filling member 20 is unlashed to enable the filling member 20 to separate from the thread 50 by pulling the second end 502 of the thread 50.

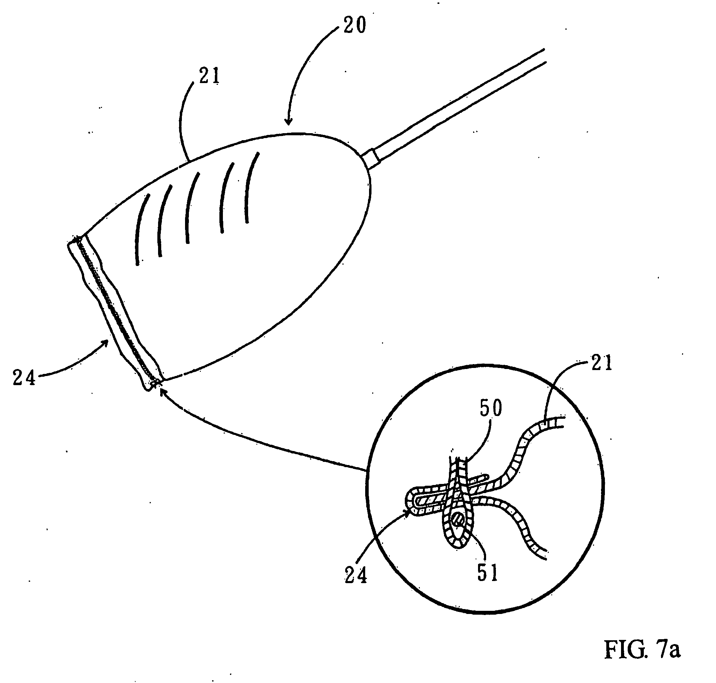

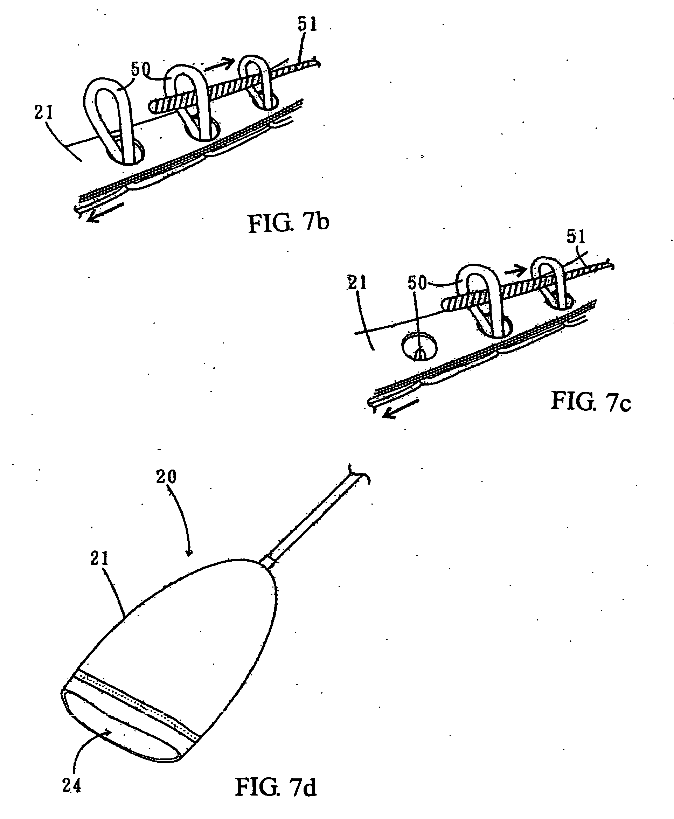

The opening 24 of the filling member 20 can be releasably fastened by sewing in conjunction with two threads 50 and 51, as illustrated in FIGS. 7a-7d. With the first thread 50, a plurality of loops are formed. These loops are joined with the flexible wall 21 of the opening 24 by sewing. The second thread 51 is put through the loops of the first thread 50. As the second thread 51 is pulled out of the loops of the first thread 50, the first thread 50 becomes separated from the flexible wall 21 of the opening 24 of the filling member 20, as illustrated in FIGS. 7b-7d. As a result, the opening 24 is unfastened. Such a fastening as described above is similar to that which is commonly used to fasten the opening of a cement or flour bag.

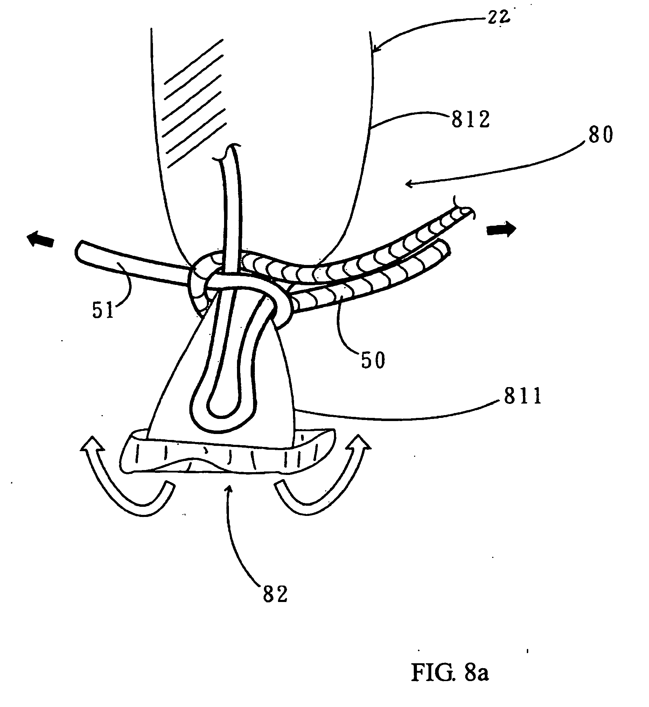

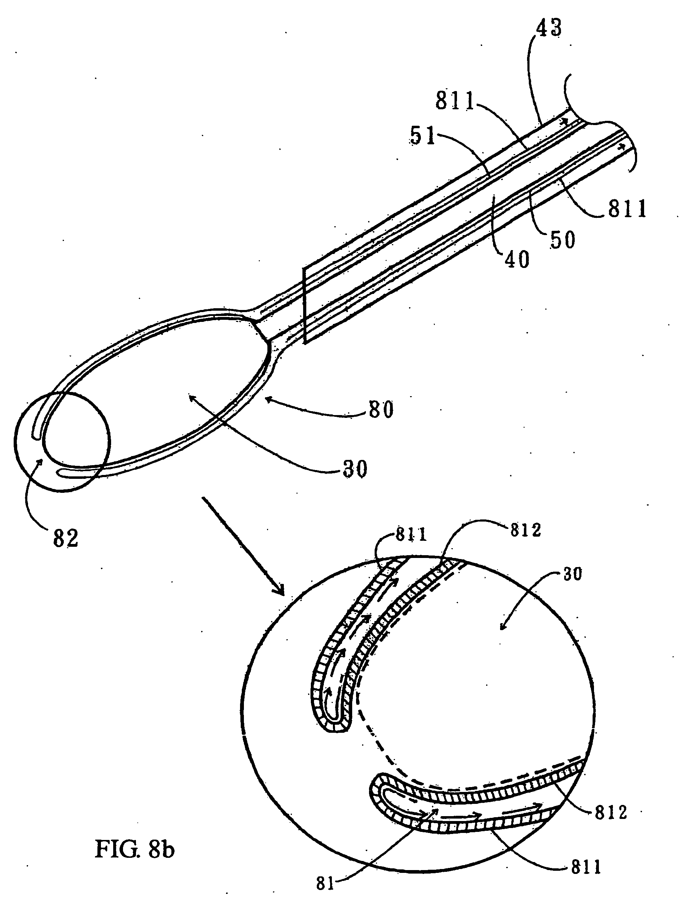

A further embodiment of the present invention is shown in FIG. 8a, and FIG. 8b, which is similar to the embodiment shown in FIGs. 1 to 2c, except that a filling member 80 is formed of a double-layer wall 81 and the first thread 51 and second thread 52 are located between an inner layer 812 and an outer layer 811 of the double-layer wall 81. As shown in FIG. 8a, a flexible and hermetic tubular wall is tied at an intermediate point thereof by the threads 50 and 51 at the beginning. The lower portion 811 of the tubular wall (will become an outer layer) is then rolled up, so that it is inside out and covering up the threads 50 and 51 and the upper portion 812 of the tubular wall (will become an inner layer). The rolled-up end of said double-layer wall 81 is provided with an opening 82 of the holding portion 22, which is lashed by the two threads 50 and 51. The opening 82 is unfastened by pulling the threads 50 and 51 the same way as shown in FIGs. 4a to 4c. As shown in FIG. 8b, the rolled-up double-layer end is retreated from the solidified medicine 30 by pulling a free end of the outer layer 811 of the double-layer wall 81, while one end of the inner layer 812 is connected to the connection tube 40 as an injection port of said holding portion 22 of the said filling member 80, whereby said solidified medicine 30 is released from said filling member 80. A working tube 43 is used to accommodate the connection tube 40, the threads 50 and 51 and the free end of the outer layer 811 of the double-layer wall 81 of the filling member 80.

The embodiments of the present invention described above are to be regarded in all respects as being illustrative and nonrestrictive. Accordingly, the present invention may be embodied in other specific forms and is limited only by the scope of the following claims.

## Claims

1. An extractable device (10) for inserting a medicinal filling into an animal tissue, said device comprising:
a filling member (20) comprising a flexible and hermetic wall (21) and provided with a holding portion (22), an injection port (23) at one end of the holding portion (22), and an opening (24) at another end of the holding portion (22); and
a pasty medicine (30) to be injected into said holding portion (22) via said injection port (23) of said filling member (20) in the wake of a process for inserting said filling member (20) into the animal tissue whereby said pasty medicine (30) solidifies in said holding portion (22) of said filling member (20); said opening of said holding portion being operable
thereby enabling said filling member (20) to be extracted from the animal tissue so as to leave only said medicine (30) in the animal tissue,
**characterised in that** said device also comprises one or more thread (50, 51), each having one end for fastening releasably said opening (24) of said holding portion (22) in such a manner that said opening (24) is leakproof, whereby said opening (24) of said holding portion (22) is unfastened at the time when the other end of said thread (50, 51) is pulled by an external force.

2. The device (10) as defined in claim 1, wherein said holding portion (22) of said filling member (20) is integrally formed by said flexible and hermetic wall (21) into a body in the form of sac, bag, or ball.

3. The device (10) as defined in claim 1, wherein said pasty medicine (30) is a mixture of a liquid and a medicinal powdered substance or medicinal granular substance.

4. The device (10) as defined in claim 1 further comprising an injection tool (60) for injecting said pasty medicine (30) into said holding portion (22) via said injection port (23).

5. The device (10) as defined in claim 4, wherein said injection tool (60) comprises a guide tube (40) and a syringe (61, 62), wherein one end (41), of said guide tube (40) is connected to said injection port (23) of said filling member (20) and another end (42) of said guide tube (40) is connected to said syringe (61, 62) in which said pasty medicine (30) is held, so that said pasty medicine (30) is able to be injected into said holding portion (22) of said filling member (20) by said syringe (61, 62) via said injection port (23) and said guide tube (40).

6. The device (10) as defined in claim 1, wherein said flexible and hermetic wall (21) is a double-layer tubular wall having one end of an inner layer thereof being provided with said injection port (23) of said holding portion (22) and having another end there of being a folded double-layer end with said opening (24) of said holding portion (22), wherein said medicine (30) is released from said filling member (20) by pulling a free end of an outer layer of the double-layer tubular wall (21) to retreat the folded double-layer end, after said opening (24) of said holding portion (22) being unfastened.

7. The device (10) as defined in claim 6, wherein said one or more thread (50, 51) is between said inner layer and said outer layer of said double-layer tubular wall (21).

8. The device (10) as defined in claim 5, wherein said flexible and hermetic wall (21) is a double-layer tubular wall having one end of an inner layer thereof being provided with said injection port (23) of said holding portion (22), and having another end thereof being a folded double-layer end with said opening (24) of said holding portion (22), wherein said medicine (30) is released from said filling member (20) by pulling a free end of an outer layer of the double-layer tubular wall to retreat the folded double-layer end, after said opening of said holding portion (22) being unfastened.

9. The device (10) as defined in claim 8, wherein said one or more thread (50, 51) is between said inner layer and said outer layer of said double-layer tubular wall (21).

## Patentansprüche

1. Ausziehbare Vorrichtung (10) zum Einbringen einer medikamentösen Füllung in ein tierisches Gewebe, wobei die genannte Vorrichtung die folgenden Merkmale umfasst:
- ein mit einer flexiblen und hermetischen Wand (21) ausgestattetes Füllelement (20), das mit einem Haltebereich (22), einer Einspritzöffnung (23) an dem einen Ende des Haltebereichs (22) und einer Öffnung (24) an dem anderen Ende des Haltebereichs (22) versehen ist; und
- ein pastöses Medikament (30) zum Einspritzen in den genannten Haltebereich (22) über die genannte Einspritzöffnung (23) des genannten Füllelements (20) als Folge eines Verfahrens zur Einführung des genannten Füllelements (20) in das tierische Gewebe, wobei sich das genannte pastöse Medikament (30) in dem genannten Haltebereich (22) des genannten Füllelements (20) verfestigt wobei die genannte Öffnung des genannte Haltebereichs betreibbar ist, so dass das genannte Füllelement (20) aus dem tierischen Gewebe extrahiert werden kann, so dass nur das genannte Medikament (30) in dem tierischen Gewebe verbleibt, **dadurch gekennzeichnet, dass** die genannte Vorrichtung weiter einen oder mehrere Fäden (50, 51) umfasst, wobei jeder jeweils ein Ende für das lösbare Verschließen der genannten Öffnung (24) des genannten Haltebereichs (22) aufweisen und zwar so, dass die genannte Öffnung (24) dicht ist, wobei die genannte Öffnung (24) des genannten Haltebereichs (22) dann gelöst wird, wenn das andere Ende des genannten Fadens (50, 51) durch eine äußere Kraft gezogen wird.

2. Vorrichtung (10) nach Anspruch
wobei der genannte Haltebereich (22) des genannten Füllelements (20) durch die genannte flexible und hermetische Wand (21) in einem Stück als Körper in Form eines Beutels, Sackes oder Balles ausgeformt ist.

3. Vorrichtung (10) nach Anspruch 1,
wobei das genannte pastöse Medikament (30) ein Gemisch aus einer Flüssigkeit und einer medikamentösen pulvrigen Substanz oder medikamentösen granulösen Substanz ist.

4. Vorrichtung (10) nach Anspruch 1,
weiter umfassend ein Einspritzwerkzeug (60) für das Einspritzen des genannten pastösen Medikaments (30) in den genannten Haltebereich (22) über die genannte Einspritzöffnung (23).

5. Vorrichtung (10) nach Anspruch 4,
wobei das genannte Einspritzwerkzeug (60) ein Führungsrohr (40) und eine Spritze (61, 62) umfasst, wobei ein Ende (41) des genannten Führungsrohrs (40) mit der genannten Einspritzöffnung (23) des genannten Füllelementes (20) verbunden ist und ein anderes Ende (42) des genannten Führungsrohrs (40) mit der genannten Spritze (61, 62), in der sich das genannte pastöse Medikament (30) befindet, verbunden ist, so dass das genannte pastöse Medikament (30) durch die genannte Spritze (61, 62) über die genannte Einspritzöffnung (23) und das genannte Führungsrohr (40) in den genannten Haltebereich (22) des genannten Füllelementes (20) eingespritzt werden kann.

6. Vorrichtung (10) nach Anspruch 1,
wobei die genannte flexible und hermetische Wand (21) eine doppelschichtige, schlauchförmige Wand ist, die ein Ende einer inneren Schicht hiervon hat, das mit der genannten Einspritzöffnung (23) des genannten Haltebereichs (22) versehen ist und die ein anderes Ende hiervon hat, das ein gefaltetes, doppelschichtiges Ende mit der genannten Öffnung (24) des genannten Haltebereichs (22) ist, worin das genannte Medikament (30) aus dem genannten Füllelement (20) abgegeben wird, indem an einem freien Ende einer äußeren Schicht der doppelschichtigen, schlauchförmigen Wand (21) gezogen wird, um das gefaltete, doppelschichtige Ende zurückzuziehen, nach dem Lösen der genannten Öffnung (24) des genannten Haltebereichs (22).

7. Vorrichtung (10) nach Anspruch 6,
wobei ein oder mehrere Fäden (50, 51) sich zwischen der genannten inneren Schicht und der genannten äußeren Schicht der genannten doppelschichtigen, schlauchförmigen Wand (21) befinden.

8. Vorrichtung (10) nach Anspruch 5,
wobei die genannte flexible und hermetische Wand (21) eine doppelschichtige, schlauchförmige Wand ist, die ein Ende einer inneren Schicht hiervon hat, das mit der genannten Einspritzöffnung (23) des genannten Haltebereichs (22) versehen ist und die ein anderes Ende hiervon hat, das ein gefaltetes, doppelschichtiges Ende mit der genannten Öffnung (24) des genannten Haltebereichs (22) ist, worin das genannte Medikament (30) aus dem genannten Füllelement (20) abgegeben wird, indem an einem freien Ende einer äußeren Schicht der doppelschichtigen, schlauchformigen Wand (21) gezogen wird, um das gefaltete, doppelschichtige Ende zurückzuziehen, nach dem Lösen der genannten Öffnung (24) des genannten Haltebereichs (22).

9. Vorrichtung (10) nach Anspruch 8,
wobei einer oder mehrere der Fäden (50, 51) sich zwischen der genannten inneren und der genannten äußeren Schicht der genannten doppelschichtigen, schlauchförmigen Wand (21) befinden.

## Revendications

1. Dispositif extractible (10) pour insérer un remplissage médicinal dans un tissu animal, ledit dispositif comprenant :
un élément de remplissage (20) comprenant une paroi flexible et hermétique (21) et doté d'une partie de support (22), d'un orifice d'injection (23) au niveau d'une extrémité de la partie de support (22) et d'une ouverture (24) au niveau d'une autre extrémité de la partie de support (22) ;
un médicament pâteux (30) destiné à être injecté dans ladite partie de support (22) via ledit orifice d'injection (23) dudit élément de remplissage (20) après un procédé pour insérer ledit élément de remplissage (20) dans le tissu animal, moyennant quoi le médicament pâteux (30) se solidifie dans ladite partie de support (22) dudit élément de remplissage (20) ; ladite ouverture de ladite partie de support pouvant être actionnée, permettant ainsi audit élément de remplissage (20) d'être extrait du tissu animal afin de ne laisser que ledit médicament (30) dans le tissu animal,
**caractérisé en ce que** ledit dispositif comprend également un ou plusieurs fils (50, 51), ayant chacun une extrémité pour fixer de manière amovible ladite ouverture (24) de ladite partie de support (22) de sorte que ladite ouverture (24) est étanche, moyennant quoi ladite ouverture (24) de ladite partie de support (22) n'est pas fixée au moment ou l'autre extrémité dudit fil (50, 51) est tirée par une force externe.

2. Dispositif (10) selon la revendication 1, dans lequel ladite partie de support (22) dudit élément de remplissage (20) est formée de manière solidaire par ladite paroi flexible et hermétique (21) dans un corps sous la forme d'un sachet, d'un sac ou d'une balle.

3. Dispositif (10) selon la revendication 1, dans lequel ledit médicament pâteux (30) est un mélange d'un liquide et d'une substance en poudre médicinale ou une substance granulaire médicinale.

4. Dispositif (10) selon la revendication 1, comprenant en outre un outil d'injection (60) pour injecter ledit médicament pâteux (30) dans ladite partie de support (22) via ledit orifice d'injection (23).

5. Dispositif (10) selon la revendication 4, dans lequel ledit outil d'injection (60) comprend un tube de guidage (40) et une seringue (61, 62), dans lequel une extrémité (41) dudit tube de guidage (40) est raccordée audit orifice d'injection (23) dudit élément de remplissage (20) et une autre extrémité (42) dudit tube de guidage (40) est raccordée à ladite seringue (61, 62), dans laquelle ledit médicament pâteux (30) est contenu, de sorte que ledit médicament pâteux (30) peut être injecté dans ladite partie de support (22) dudit élément de remplissage (20) par ladite seringue (61, 62) via ledit orifice d'injection (23) et ledit tube de guidage (40).

6. Dispositif (10) selon la revendication 1, dans lequel ladite paroi flexible et hermétique (21) est une paroi tubulaire à deux couches ayant une extrémité de sa couche interne qui est prévue avec un orifice d'injection (23) de ladite partie de support (22), et ayant son autre extrémité qui est une extrémité pliée à deux couches avec ladite ouverture (24) de ladite partie de support (22), dans lequel ledit médicament (30) est libéré dudit élément de remplissage (20) en tirant sur une extrémité libre d'une couche externe de la paroi tubulaire à double couche (21) pour faire reculer l'extrémité pliée à deux couches, après que ladite ouverture (24) de ladite partie de support (22) a été débloquée.

7. Dispositif (10) selon la revendication 6, dans lequel lesdits un ou plusieurs fils (50, 51) sont entre ladite couche interne et ladite couche externe de ladite paroi tubulaire à deux couches (21).

8. Dispositif (10) selon la revendication 5, dans lequel ladite paroi flexible et hermétique (21) est une paroi tubulaire à deux couches, ayant une extrémité de sa couche interne qui est prévue avec ledit orifice d'injection (23) de ladite partie de support (22), et ayant son autre extrémité qui est une extrémité pliée à deux couches avec ladite ouverture (24) de ladite partie de support (22), dans lequel ledit médicament (30) est libéré dudit élément de remplissage (20) en tirant sur une extrémité libre d'une couche externe de la paroi tubulaire à deux couches pour faire reculer l'extrémité pliée à deux couches, après que ladite ouverture de ladite partie de support (22) a été débloquée.

9. Dispositif (10) selon la revendication 8, dans lequel lesdits un ou plusieurs fils (50, 51) sont entre ladite couche interne et ladite couche externe de ladite paroi tubulaire à deux couches (21).
